# EUROPEAN PATENT SPECIFICATION

(11) **EP 1 201 214 B1**
(45) Date of publication and mention of the grant of the patent: **08.09.2004**
(21) Application number: 01309040.2
(22) Date of filing: 24.10.2001
(51) Int. Cl.: A61F 13/551, A61F 13/15

(54) **Disposable pull-on diaper**
Wegwerfhöschen
Couche-culotte jetable

(30) Priority: 25.10.2000 JP 2000325827
(43) Date of publication of application: 02.05.2002
(73) Proprietor: Uni-Charm Corporation, Shikokuchuo-shi, Ehime-ken (JP)
(72) Inventor: Suzuki, Seiji, c/o Technical Center, Mitoyo-gun, Kagawa-ken 769-1602 (JP)
(74) Representative: Parry, Christopher Stephen

(56) References cited:
- EP-A- 0 890 351
- EP-A- 1 092 405
- EP-A- 1 121 917
- EP-A- 1 121 918
- DATABASE WPI Derwent Publications Ltd., London, GB; AN 1997-530468 XP002219051 & JP 1997 253123 A (UNI-CHARM KK)

## Description

This invention relates to a disposable pull-on diaper provided with tape fasteners used to fasten the diaper in a rolled up state for its disposal.

Japanese Patent Application Publication Nos. 1997-253123A and 1997-253124A describe disposable pull-on diapers comprising a liquid-pervious topsheet, a liquid-impervious backsheet and a liquid-absorbent core disposed between these two sheets so as to configure front and rear waist regions opposed to each other and a crotch region positioned between these waist regions wherein, in the rear waist region, tape fastener(s) is(are) attached to the outer surface of the backsheet so that the used diaper may be fastened in a rolled up state for its disposal.

In the diaper described in Japanese Patent Application Publication No. 1997-253123A, the tape fastener is formed with a single tape strip extending in a waist-surrounding direction and bonded to the diaper at its central zone in the longitudinal direction. The remaining right and left side portions of the tape strip are folded up so that these portions may be folded right- and leftward in the waist-surrounding direction. The right and left side portions are provided with a bonding zone so as to keep the folded up state of the diaper

In the diaper described in Japanese Patent Application Publication No. 1997-253124A, the tape fasteners are formed with a pair of strips of adhesive tape extending in parallel to each other in the waist-surrounding direction and spaced apart from each other in the up and down direction of the diaper.

For disposal of the diaper disclosed in the Publications after use, the diaper is rolled up with the front waist region inside from the opposite side edge portions thereof in a transverse direction toward the tape fastener(s) and then the tape fastener(s) wound round the diaper is or are anchored on the outer surface of the backsheet by means of pressure-sensitive adhesive. The rolled up diaper is fastened round by the tape fastener(s) in this manner and held in rolled up state.

The diaper of prior art disclosed in these Publications requires its user to roll up the diaper from its both side edges toward the tape fastener(s) and then to wind the tape fastener(s) around the rolled up diaper. With a consequence, much time and labor are required for the operation of rolling up the diaper and for the operation of winding the tape fastener(s) around this rolled up diaper. In addition, it is impossible for such diaper of prior art to close the waist-opening using the tape fasteners and there is always an anxiety that excretion and/or its odor might leak from the waist-opening.

It is an object of this invention to provide a disposable pull-on diaper adapted to be easily rolled up and fastened round for its disposal after use without any apprehension that excretion and/or its odor might leak from the waist-opening and the pair of leg-openings.

According to this invention, there is provided a disposable pull-on diaper comprising front and rear waist regions opposed to each other and a crotch region positioned between these waist regions, the front and rear waist regions being connected together along transversely opposite side edge portions thereof to define a waist-opening and a pair of leg-openings with a pair of tape fasteners adapted to fasten the diaper in rolled up state attached to the outer surface of the diaper and an anchoring means formed on inner surfaces facing of the tape fasteners facing opposed to the outer surface of the diaper.

The disposable pull-on type diaper further comprises the tape fasteners being provided adjacent the transversely opposite side edge portions in one of the front and rear waist regions so as to extend in a longitudinal direction wherein each of the tape fasteners has upper end portions lying adjacent the waist-opening and lower end portions lying adjacent the leg-openings, only the lower end portions of the tape fastners being bonded to the diaper by means of bonding zones extending in a waist-surrounding direction adjacent a peripheral edge of the leg-openings wherein the bonding zones obliquely extending inward in the waist-surrounding direction from the transversely opposite side edge portions toward the upper end portions.

According to one preferred embodiment of this invention, the lower end portions of each of the tape fasteners are positioned inwardly in the waist-surrounding direction with respect to the upper end portions of each of the tape fasteners, and each of the tape which lies obliquely extending from its lower end portions toward its upper end portions so as to be gradually spaced away each other.

According to another preferred embodiment of this invention, elastically stretchable members associated with the leg-openings extending in the leg-surrounding direction are attached under extension to the peripheral edge portions of the leg-openings and the bonding zones overlap at least partially the elastically stretchable members associated with the leg-openings.

According to still another preferred embodiment of this invention, the anchoring means are formed with one of pressure-sensitive adhesive agents applied on the tape fasteners and hook members attached to the tape fasteners and release sheets adapted for temporarily retaining the tape fasteners are attached to the outer surface of the diaper adjacent the transversely opposite side edge portions.
Fig. 1 is a perspective view showing a disposable pull-on diaper as viewed from the side of the rear waist region and as partially broken away;
Fig. 2 is a sectional view taken along a line A - A in Fig. 1;
Fig. 3 is a sectional view taken along a line B - B in Fig. 1;
Fig. 4 is a perspective view showing the diaper of Fig. 1 as having been rolled up for its disposal;
Fig. 5 is a perspective view showing the diaper of Fig. 1 as having been rolled up for its disposal;
Fig. 6 is a perspective view showing another embodiment of the diaper as partially broken away; and
Fig. 7 is a perspective view showing the diaper of Fig. 6 as having been rolled up for its disposal.

Details of the disposable pull-on diaper according to this invention will be more fully understood from the description given hereunder with reference to the accompanying drawings.

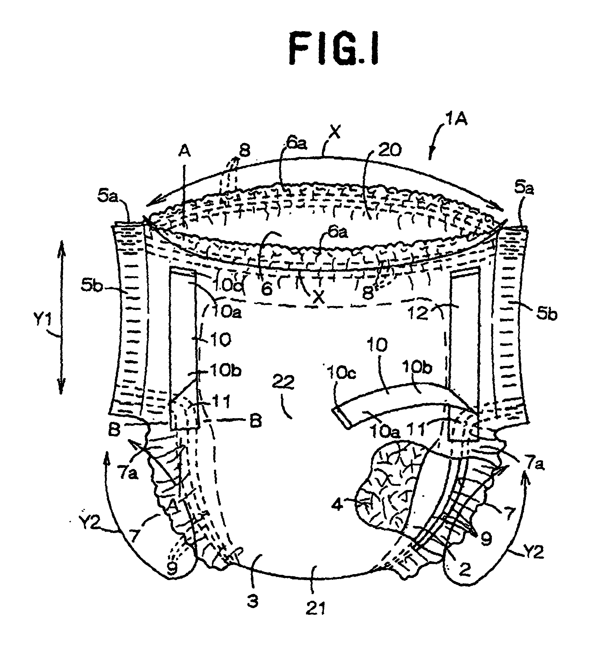

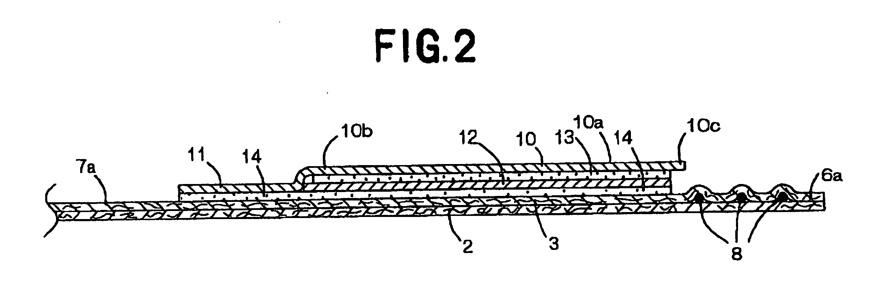

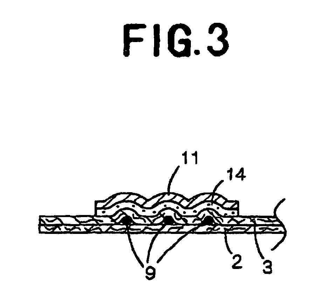

Fig. 1 is a perspective view showing a disposable pull-on diaper 1A as viewed from the side of a rear waist region 22 and as partially broken away, Fig. 2 is a sectional view taken along a line A - A in Fig. 1, and Fig. 3 is a sectional view taken along a line B - B in Fig. 1. In Fig. 1, one of tape fasteners 10 is shown as being separated from a release sheet 12. Referring to Fig. 1, a waist-surrounding direction is indicated by an arrow X, a longitudinal direction is indicated by an arrow Y1 and a leg-surrounding direction is indicated by an arrow Y2. Surfaces of top- and backsheets 2, 3, the tape fasteners 10, the release sheets 12 and the others which face a core 4 will be referred to herein as "inner surfaces" and outer surfaces of these components 2, 3, 10, 12 which do not face the core 4 will be referred to herein as "outer surfaces".

The diaper 1A is basically composed of the liquid-pervious topsheet 2, the liquid-impervious backsheet 3 and the liquid-absorbent core 4 disposed between these sheets 2, 3 and entirely covered with and bonded to tissue paper (not shown). The core 4 is bonded to the inner surfaces of the top- and backsheets 2, 3 with the tissue paper therebetween.

The diaper 1A is composed of front and rear waist regions 20, 22 opposed to each other and a crotch region 21 positioned between these waist regions 20, 22. The front and rear waist regions 20, 22 are overlaid and bonded together along transversely opposite side edges 5a, 5b thereof extending in a longitudinal direction so as to define a waist-opening 6 and a pair of leg-openings 7.

Along a peripheral edge portion 6a of the waist-opening 6, an elastic member 8 comprising a plurality of elastic elements expandable in the waist-surrounding direction is disposed between the top- and backsheets 2, 3 and bonded under extension to the inner surfaces of these sheets 2, 3. Along a peripheral edge portion 7a of each leg-opening 7, an elastic member 9 comprising a plurality of elastic elements expandable in a leg-surrounding direction is disposed between the top- and backsheets 2, 3 and bonded under extension to the inner surfaces of the sheets 2, 3. Referring to Fig. 1, a plurality of gathers are formed along the peripheral edge portions 6a, 7a of the waist- and leg-openings 6, 7 as the elastic members 8, 9 contract themselves.

In the transversely opposite side edge portions 5b of the rear waist region 22, a pair of tape fasteners 10 are attached to the outer surface of the backsheet 3 so that the used diaper 1A may be fastened by these tape fasteners 10 in rolled up state. The tape fasteners 10 are made of a flexible but non-expandable plastic sheet. These tape fasteners 10 extend in parallel to the transversely opposite side edge portions 5b in the longitudinal direction.

Each of the tape fasteners 10 has an upper end portion 10a lying adjacent the waist-opening 6 and a lower end portion 10b lying adjacent the associated leg-opening 7. Each of the fastener tapes 10 has its lower end portion 10b bonded to the outer surface of the backsheet 3 through a bonding zone 11 extending in the waist-surrounding direction. Each of the tape fasteners 10 is coated on its inner surface with a pressure-sensitive adhesive 13. The upper end portion 10a of one of the tape fasteners 10 is formed with a grip 10c not coated with the pressure-sensitive adhesive 13.

The bonding zones 11 overlap the elastic member 9 associated with the leg-opening 6 and obliquely extend inwardly from the transversely opposite side edge portions 5b of the rear waist region 22 toward the upper end portions 10a in the waist-surrounding direction. In the bonding zones 11, the inner surface of each of the tape fasteners 10 is bonded to the outer surface of the backsheet 3 by means of adhesive 14. While the bonding zones 11 overlap completely with the elastic member 9 associated with the leg-openings, the desired effect may be achieved by placing the bonding zones 11 so as to overlap with at least one of the elastic elements of the elastic member 9 associated with the leg-opening.

The release sheets 12 for temporary fixation of the tape fasteners 10 are disposed between the tape fasteners 10 and the backsheet 3. The release sheets 12 are made of a flexible plastic sheet and its inner surfaces thereof are bonded to the outer surface of the backsheet 3 by means of adhesives 14. Each of the tape fasteners 10 is temporarily fixed to the outer surface of the release sheets 12 by means of the pressure-sensitive adhesive 13. Hot melt adhesive is preferably used as the adhesives 14. The tape fasteners 10 as well as the release sheets 12 may be firmly bonded to the backsheet 3 using a technique of welding, instead of using adhesive.

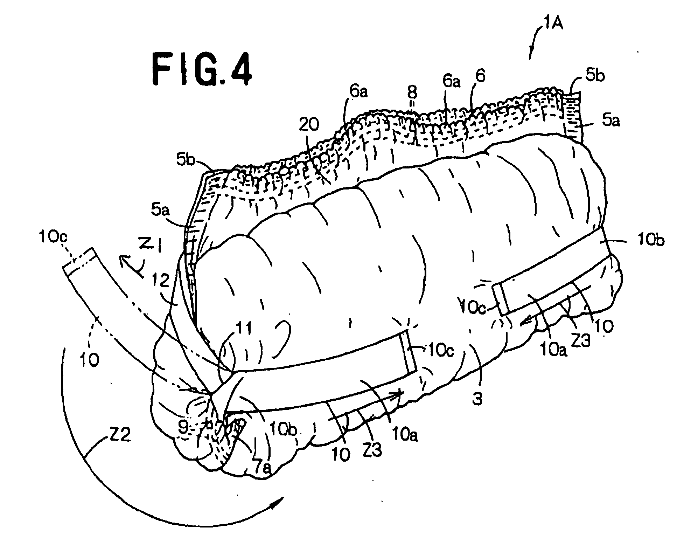

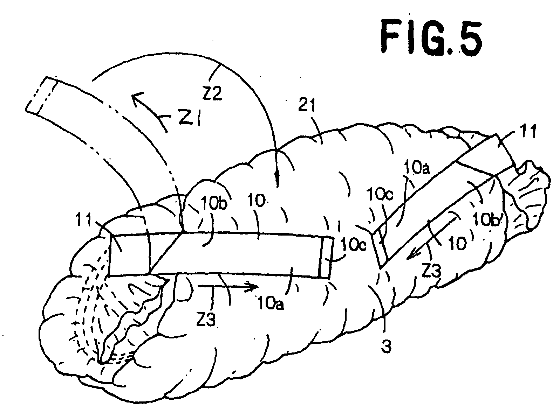

Figs. 4 and 5 are perspective views showing the diaper 1A of Fig. 1 as having been rolled up for its disposal. In Figs. 4 and 5, a state of one of the tape fasteners 10 peeled off from the associated release sheets 12 is indicated by two-dots chain lines. Referring to Fig. 4, the diaper 1A has been rolled up in the longitudinal direction starting from the crotch region 21 toward the peripheral edge portion 6a of the waist-opening 6 with the front waist region 20 inside. Referring to Fig. 5, on the contrary, the diaper 1A has been rolled up in the longitudinal direction starting from the peripheral edge portion 6a of the waist-opening 6 toward the crotch region 21 with keeping the front waist region 20 inside. With the diaper 1A having rolled up in such manner, each of the tape fasteners 10 is anchored on the outer surface of the backsheet 3 by means of pressure-sensitive adhesive 13.

To fasten the rolled up diaper 1A with the tape fasteners 10, the tape fasteners 10 are peeled off from the release sheets 12 respectively by holding the grips 10c of the fasteners 10 respectively with the fingers. Then, the tape fasteners 10 are pulled around the rolled up diaper in a direction indicated by an arrow Z2 and pressed against the outer surface of the backsheet 3.

The diaper 1A has the bonding zones 11 extending obliquely, so the respective tape fasteners 10 extends outward obliquely as indicated by two-dots chain lines in Figs. 4 and 5 as the tape fasteners 10 are peeled off in the direction Z1. In the case of the diaper 1A according to this embodiment, once the tape fasteners 10 have been peeled off from the respective release sheets 12, the tape fasteners 10 can be easily anchored on the rolled up diaper 1A merely by guiding the tape fasteners 10 in the direction indicated by the arrow Z2 without need to change the directions of the respective tape fasteners 10 and then pressing the tape fasteners 10 against the outer surface of the backsheet 3.

In case of the diaper 1A, the bonding zones 11 are overlapped with the respective elastic member 9 associated with the leg-opening 7 so that an elongational force is exerted on the elastic member 9 associated with the leg-opening 7 if the tape fasteners are stretched so as to be spaced apart from the diaper 1A. Pulling of the tape fasteners 10 of the diaper 1A in a direction indicated by an arrow Z3 may stretch the elastic members 9 associated with the leg-opening 7 and the peripheral edge portion 7a of the leg-opening 7. Anchoring of the tape fasteners 10 of the diaper 1A on the outer surface of the backsheet 3 with the elastic member 9 associated with the leg-opening 7 as well as the peripheral edge portion 7a of the respective leg-opening 7 held under extension in a direction indicated by an arrow line Z3 will close the leg-opening 7 by stretched peripheral edge portion 7a.

According to this embodiment, it is ensured that the diaper 1A is fastened by the respective tape fasteners 10 in its rolled up state and the leg-opening 7 is reliably maintained in closed state by the peripheral edge portions 7a of the respective leg-opening 7. Consequently, there is no anxiety that the leg-opening 7 closed in this manner might be unintentionally reopened and excretion and/or its odor might leak from the leg-opening 7. Once the diaper 1A has been rolled as seen in Fig. 5, the peripheral edge portion 6a of the waist-opening 6 is confined within the rolled up diaper 1A and therefore there is no anxiety that the waist-opening 6 might be unintentionally exposed to outside and excretion and/or its odor might leak from the waist-opening 6.

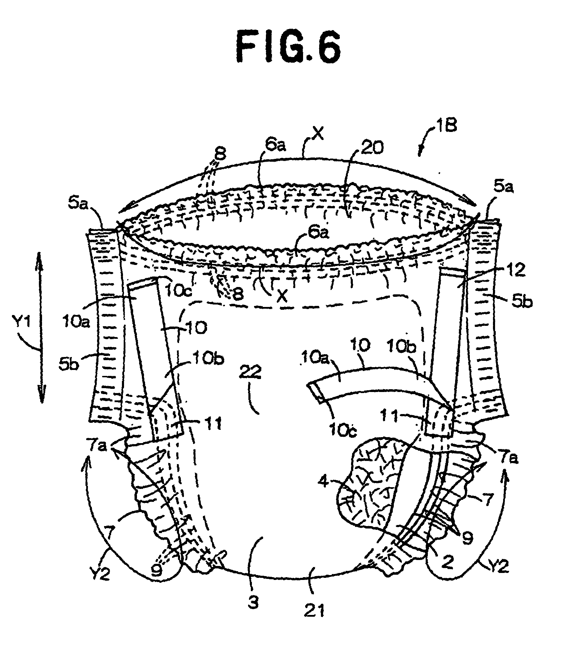

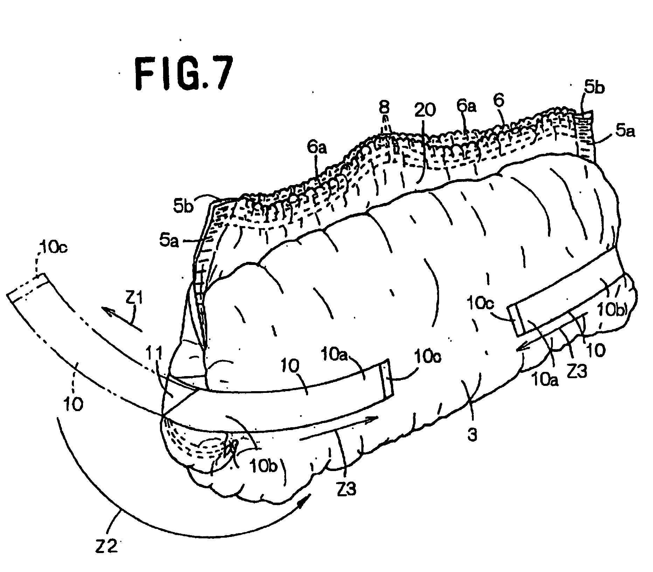

Fig. 6 is a perspective view of the diaper 1B according to another embodiment of this invention as viewed as partially broken away and Fig. 7 is a perspective view of the diaper 1B in Fig. 6 as having been rolled up for its disposal. Fig. 6 illustrates the diaper 1B as viewed from the side of the rear waist region 22 with one of the tape fasteners 10 having been peeled off from the associated release sheet 12. Fig. 7 illustrates the diaper 1B with one of the tape fasteners 10 having been peeled off from the associated release sheet 12 and turned up as shown by two-dots chain lines.

Configuration of the diaper 1B is basically similar to that of the diaper 1A illustrated in Fig. 1 and detailed description thereof will be eliminated. The diaper 1B of Fig. 6 is distinguished from the diaper 1A of Fig. 1 in that the lower end portion 10b of each of the tape fasteners 10 is positioned more inwardly with respect to the upper end portion 10a in the waist-surrounding direction and the respective tape fasteners 10 lies obliquely extending so that these two tape fasteners 10 are gradually spaced away each other from the lower end portions 10b toward the upper end portions 10a.

As is illustrated in Fig. 7, the diaper 1B has been rolled up in the longitudinal direction from the crotch region 21 toward the peripheral edge portion 6a of the waist-opening 6 with the front waist region 20 inside. When the diaper 1B has been rolled up, the tape fasteners 10 are anchored on the outer surface of the backsheet 3 by means of pressure-sensitive adhesive (not shown).

In the diaper 1B according to this alternative embodiment, the pair of tape fasteners 10 obliquely extend so that these tape fasteners 10 are gradually spaced away each other from the lower end portions 10b toward the upper end portions 10a and the bonding zones 11 obliquely extend inwardly in the waist-surrounding direction from the transversely opposite side edge portions 5 toward the upper end portions 10a. Therefore, peeling off of the tape fasteners 10 in the direction Z1 causes the tape fasteners 10 to extend laterally as indicated by two-dots chain lines in Fig. 7. After the tape fasteners 10 have been peeled off, these tape fasteners 10 may be merely turned up in the direction Z2 without need to redirect the tape fasteners 10 and anchored on the outer surface of the backsheet 3.

The diaper 1B is similar to the diaper 1A of Fig. 1 in that the tape fasteners 10 may be pulled in the direction Z3 to stretch the elastic member 9 associated with the leg-opening 7 and the peripheral edge portion 7a of the respective leg-openings 7. In this way, the diaper 1B is held by the respective tape fasteners 10 in rolled up state in which the leg-opening 7 is maintained in closed state under the peripheral edge portion 7a stretched in the direction Z3.

For disposal of the diaper 1B, it is also possible to roll the diaper up starting from the peripheral edge portion 6a of the waist-opening 6 toward the crotch region 21 in the longitudinal direction with the front waist region 20 inside.

The tape fasteners 10 may be formed, in addition to a non-stretchable plastic sheet, with an elastically stretchable elastomer such as synthetic or natural rubber or such elastomer bonded under extension to nonwoven fabric.

With the backsheet 3 made of fibrous nonwoven fabric, it is possible to attach a hook member serving as a component of so-called mechanical fastener to the inner surface of each of the tape fastener 10, instead of coating the inner surfaces of the tape fasteners 10 with pressure-sensitive adhesive 13. In this case, the hook member is engaged with component fibers of the nonwoven fabric so that the tape fasteners 10 may be reliably anchored on the outer surface of the backsheet 3 of the rolled up diapers 1A, 1B. In the case using the hook members attached to the tape fasteners 10, it is possible to replace the release sheets 12 by loop members which may be attached to the outer surface of the backsheet 3 in the vicinity of transversely opposite side edge portion 5b of the rear waist region 22. It is also possible to attach the tape fasteners 10 as well as the release sheets 12 to the outer surface of the backsheet 3 in the vicinity of the transversely opposite side edges 5a of the front waist region 20.

The topsheet 2 may be formed with a liquid-pervious hydrophilic sheet or a nonwoven fabric or porous plastic film. The backsheet 3 may be formed with a hydrophobic nonwoven fabric, a liquid-impervious plastic film or a laminated sheet of hydrophobic nonwoven fabric with a plastic film. It is also possible to use for the backsheet 3, a nonwoven composite fabric made of melt blown process having a highly water-resistant-property which are sandwiched by two layers of spun bond fibrous nonwoven fabric having a high strength and flexibility.

The nonwoven fabric may be selected from a group including spun lace-, needle punch-, melt blown-, thermal bond-, spun bond-, chemical bond- and air through-nonwoven fabric. Component fiber of the nonwoven fabric may be selected from a group including polyolefine-, polyester- and polyamide-based fibers and polyethylene/polypropylene or polyethylene/polyester core-sheath type conjugated fiber and side-by-side-type conjugated fiber.

The core 4 is a mixture of fluff pulp, high absorption polymer grains and thermoplastic synthetic resin fiber compressed to a desired thickness. The high absorption polymer may be selected from a group including starch-, cellulose-based polymer and synthetic polymer.

Bonding of the top- and backsheets 2, 3, the core 4 as well as attachment of the elastic members 8, 9 may be carried out using suitable adhesive such as hot melt adhesive or a technique of welding such as sonic-sealing or heat-sealing.

For disposal of the disposable pull-on diaper according to this invention after use, the diaper may be rolled up in the longitudinal direction starting from the crotch region toward the waist-opening or rolled up in the longitudinal direction starting from the peripheral edge portion of the waist-opening toward the crotch region, the tape fasteners may be peeled off from the respective release sheets, then guided around the rolled up diaper without need to change their directions and anchored on the outer surface of the rolled up diaper. In this manner, operation of rolling up the used diaper as well as operation of anchoring the tape fasteners on the rolled up diaper can be carried out more easily than in the conventional diaper.

The diaper is reliably held by the tape fasteners in rolled up state with the leg-openings well maintained by the stretched peripheral edge portions thereof. In this way, there is no anxiety that the leg-openings might be unintentionally reopened and excretion and/or its odor might leak from the leg-openings.

When the diaper is rolled up starting from the peripheral edge portion of the waist-opening toward the crotch region in the longitudinal direction, the peripheral edge portion of the waist-opening is confined within the rolled up diaper and therefore it is not apprehended that the waist-opening might be exposed and excretion and/or its odor might leak from the waist-opening.

## Claims

1. A disposable pull-on diaper (1A) comprising front and rear waist regions (20, 22) opposed to each other and a crotch region (21) positioned between these waist regions (20, 22), said front and rear waist regions (20, 22) being connected together along transversely opposite side edge portions (5a, 5b) thereof to define a waist-opening (6) and a pair of leg-openings (7) with a pair of tape fasteners (10) adapted to fasten said diaper (1A) in rolled up state attached to an outer surface of said diaper (1A) and an anchoring means formed on inner surfaces of said tape fasteners facing opposed to the outer surface of said diaper, wherein:
said tape fasteners (10) are provided adjacent said transversely opposite side edge portions (5a, 5b) in one of said front and rear waist regions (20, 22) so as to extend in a longitudinal direction wherein said tape fasteners (10) respectively have upper end portions (10a) lying adjacent said waist-opening (6) and lower end portions (10b) lying adjacent said leg-openings (7), **characterized in that** only said lower end portions (10b) of said tape fasteners (10) are bonded to said diaper by means of bonding zones (11) extending in a waist-surrounding direction adjacent a peripheral edge of said leg-openings (7) wherein said bonding zones (11) obliquely extend inwardly in said waist-surrounding direction from said transversely opposite side edge portions (5a, 5b) toward said upper end portions.

2. The disposable pull-on diaper according to Claim 1, wherein said lower end portions (10b) of respective said tape fasteners (10) are positioned inwardly in said waist-surrounding direction with respect to said upper end portions (10a) of respective said tape fasteners (10) which obliquely extend from their lower end portions (10b) toward their upper end portions (10a) so as to be gradually spaced away from each other.

3. The disposable pull-on diaper according to Claim 1, wherein elastically stretchable members (9) associated with said leg-openings (7) extending in said leg-surrounding direction are attached under extension to the peripheral edge portions (7a) of respective said leg-openings (7) and said bonding zones (11) are at least partially overlapped with said elastically stretchable members (9) associated with said leg-openings (7).

4. The disposable pull-on diaper according to Claim 1, wherein said anchoring means are formed with pressure-sensitive adhesives (13) applied on said tape fasteners (10) or hook members and release sheets adapted for temporarily retaining said tape fasteners (10) are attached to the outer surface of said diaper (1A) adjacent said transversely opposite side edge portions.

## Patentansprüche

1. Wegwerf-Windel (1A) zum Anziehen, umfassend einander gegenüberliegende vordere und hintere Taillenbereiche (20, 22) und einen zwischen diesen Taillenbereichen (20, 22) liegenden Schrittbereich (21), wobei der vordere und der hintere Taillenbereich (20, 22) entlang einander in Querrichtung gegenüberliegender Seitenkantenabschnitte (5a, 5b) miteinander verbunden sind und so eine Taillenöffnung (6) und ein Paar von Beinöffnungen (7) definieren, deren letzteres ein Paar von Befestigungsstreifen (10) aufweist, das zur Befestigung der Windel (1A) im zusammengerollten Zustand ausgelegt ist und an einer äußeren Oberfläche der Windel (1A) und einer auf inneren Oberflächen der Befestigungsstreifen gebildeten und gegen die äußere Oberfläche der Windel weisenden Verankerungseinrichtung angebracht ist, wobei:
die Befestigungsstreifen (10) an den einander in Querrichtung gegenüberliegenden Seitenkantenabschnitten (5a, 5b) in dem vorderen oder dem hinteren Taillenbereich (20, 22) angeordnet sind und sich so in Längsrichtung erstrecken, wobei die Befestigungsstreifen (10) jeweils an der Taillenöffnung (6) anliegende obere Endabschnitte (10a) und an den Beinöffnungen (7) anliegende untere Endabschnitte (10b) aufweisen,
**gekennzeichnet dadurch, daß** nur die unteren Endabschnitte (10b) der Befestigungsstreifen (10) mit der Windel durch Verbindungsbereiche (11) verbunden sind, die sich entlang einer die Taille umgebenden Richtung erstrecken und an einer äußeren Kante der Beinöffnungen (7) anliegen, wobei die Verbindungsbereiche (11) schräg nach innen in der die Taille umgebenden Richtung von den einander in Querrichtung gegenüberliegenden Seitenkantenabschnitten (5a, 5b) zu den oberen Endabschnitten verlaufen.

2. Wegwerf-Windel zum Anziehen nach Anspruch 1, wobei die unteren Endabschnitte (10b) der jeweiligen Befestigungsstreifen (10) in der die Taille umgebenden Richtung nach innen bezüglich der oberen Endabschnitte (10a) der Befestigungsstreifen (10) angeordnet sind, wobei letztere schräg von ihren unteren Endabschnitten (10b) zu ihren oberen Endabschnitten (10a) verlaufen und so einen graduellen Abstand voneinander aufweisen.

3. Wegwerf-Windel zum Anziehen nach Anspruch 1, wobei den Beinöffnungen (7) zugeordnete und in der die Beine umgebenden Richtung verlaufende elastisch dehnbare Elemente (9) unter Zug an den äußeren Kantenabschnitten (7a) der jeweiligen Beinöffnungen (7) befestigt sind und die Verbindungsbereiche (11) mindestens teilweise von den den Beinöffnungen (7) zugeordneten elastisch dehnbaren Elementen (9) überlappt werden.

4. Wegwerf-Windel zum Anziehen nach Anspruch 1, wobei die Verankerungseinrichtung durch auf dem Befestigungsstreifen (10) aufgebrachte druckempfindliche Klebstoffe (13) oder durch Hakenelemente gebildet ist und wobei Ablösestreifen zur zeitweiligen Aufnahme der Befestigungsstreifen (10) an der äußeren Oberfläche der Windel (1A) anliegend an den einander in Querrichtung gegenüberliegenden Seitenkantenabschnitten angebracht sind.

## Revendications

1. Couche jetable à enfiler (1A) comprenant des régions de ceinture avant et arrière (20, 22) opposées l'une à l'autre et une région d'entre-jambes (21) agencée entre ces régions de ceinture (20, 22), lesdites régions de ceinture avant et arrière (20, 22) étant attachées ensemble le long de parties (5a, 5b) de leurs bords latéraux opposés transversalement pour définir une ouverture (6) de ceinture et une paire d'ouvertures (7) de jambes, une paire de bandes de fixation (10) aptes à attacher ladite couche (1A) à l'état enroulé étant fixées à une surface externe de ladite couche (1A), et un moyen d'ancrage étant formé sur des surfaces internes desdites bandes de fixation qui font face à la surface externe de ladite couche, dans laquelle :
lesdites bandes de fixation (10) sont disposées adjacentes auxdites parties (5a, 5b) des bords latéraux opposés transversalement dans l'une des régions de ceinture avant et arrière (20, 22) de manière à s'étendre dans une direction longitudinale, lesdites bandes de fixation (10) comportant des parties d'extrémité supérieures (10a) adjacentes à ladite ouverture (6) de ceinture et des parties d'extrémité inférieures (10b) adjacentes auxdites ouvertures (7) de jambes respectivement,
**caractérisée en ce que** seules lesdites parties d'extrémité inférieures (10b) desdites bandes de fixation (10) sont attachées à ladite couche au moyen de zones d'attache (11) qui s'étendent dans une direction entourant la ceinture et sont adjacentes chacune à un bord périphérique de l'une desdites ouvertures (7) de jambes, lesdites zones d'attache (11) s'étendant en oblique vers l'intérieur dans ladite direction entourant la ceinture à partir desdites parties (5a, 5b) de bords latéraux opposées transversalement vers lesdites parties d'extrémité supérieures.

2. Couche jetable à enfiler selon la revendication 1, dans laquelle lesdites parties (10b) d'extrémité inférieures desdites bandes de fixation respectives (10) sont positionnées vers l'intérieur, dans ladite direction entourant la ceinture, par rapport auxdites parties (10a) d'extrémité supérieures desdites bandes de fixation respectives (10) qui s'étendent en oblique à partir de leurs parties (10b) d'extrémité inférieures vers leurs parties (10a) d'extrémité supérieures de manière à être graduellement espacées l'une de l'autre.

3. Couche jetable à enfiler selon la revendication 1, dans laquelle des éléments étirables élastiquement (9), associés auxdites ouvertures (7) de jambes et s'étendant chacun dans ladite direction entourant les jambes, sont attachés en extension aux parties (7a) des bords périphériques desdites ouvertures (7) de jambes, et lesdites zones d'attache (11) sont au moins partiellement recouvertes par lesdits éléments étirables élastiquement (9) associés auxdites ouvertures (7) de jambes.

4. Couche jetable à enfiler selon la revendication 1, dans laquelle ledit moyen d'ancrage comporte des adhésifs (13) sensibles à la pression, appliqués sur lesdites bandes de fixation (10), ou en variante des éléments à crochets, et des feuilles antiadhésives aptes à retenir temporairement lesdites bandes de fixation (10) sont attachées à la surface externe de ladite couche (1A) en étant adjacentes auxdites parties des bords latéraux opposés transversalement.
